# EUROPEAN PATENT SPECIFICATION

(11) **EP 4 257 970 B1**
(45) Date of publication and mention of the grant of the patent: **10.09.2025**
(21) Application number: 23164335.4
(22) Date of filing: 27.03.2023
(51) Int. Cl.: G01N 30/06

(54) **METHOD FOR SIMULTANEOUS ANALYSIS OF PROHIBITED DRUGS AND METABOLITES THEREOF IN FOOD BY GAS CHROMATOGRAPHY-MASS SPECTROMETRY**
VERFAHREN ZUR GLEICHZEITIGEN ANALYSE VERBOTENER ARZNEIMITTEL UND METABOLITEN DAVON IN LEBENSMITTELN DURCH GASCHROMATOGRAPHIE-MASSENSPEKTROMETRIE
PROCÉDÉ D'ANALYSE SIMULTANÉE DE MÉDICAMENTS INTERDITS ET DE LEURS MÉTABOLITES DANS DES ALIMENTS PAR CHROMATOGRAPHIE EN PHASE GAZEUSE-SPECTROMÉTRIE DE MASSE

(30) Priority: 05.04.2022 KR 20220042014
(43) Date of publication of application: 11.10.2023
(73) Proprietor: Korea Institute of Science and Technology, Seoul 02792 (KR)
(72) Inventor: SON, Junghyun, 02792 Seoul (KR); LEE, Yejin, 02792 Seoul (KR); JEON, Seongeun, 02792 Seoul (KR); PARK, Hana, 02792 Seoul (KR); CHO, Yoeseph, 02792 Seoul (KR)
(74) Representative: advotec.

(56) References cited:
- CN-B- 104 897 842
- KR-B1- 100 519 033
- US-B2- 9 891 199

## Description

### [Technical Field]

The present disclosure relates to a method for simultaneous analysis of prohibited drugs and metabolites thereof, particularly to a method for simultaneous analysis of prohibited drugs and metabolites thereof, which is capable of accurately detecting unintentional doping of prohibited drugs in various food matrices.

### [Background Art]

Unintentional doping refers to a positive result in doping tests due to the accidental ingestion of drugs prohibited by the World Anti-Doping Agency by athletes without knowing. Food is known as the main cause of unintentional doping. Particularly, it occurs often due to the ingestion of meat, owing to the growth-promoting adjuvants used during stock raising or the anabolic steroids included in bull meat. Despite the incessant issues of unintentional doping, there is no preventive measure for protecting athletes.

Because it is very difficult to prove a positive result in doping tests due to food contamination, athletes should be informed of the contamination of foodstuff before taking food. Accordingly, it is necessary to protect the athletes by screening test for food in advance.

At present, detection of prohibited drugs included in food is possible for only one food matrix in many cases, and few researches have been conducted on a method for simultaneous analysis for a variety of food matrices. Accordingly, development of an optimized method for pretreatment and analysis of various foods is necessary to protect athletes and prevent unintentional doping due to ingestion of food contaminated with prohibited drugs.

### [References of Related Art]

### Patent Documents]

Korean Patent Registration Publication No. 10-1188183. Korean Patent Publication KR100519033 describes a method for detecting phytoestrogens in herbal medicines using a gas chromatography-mass spectrometer, including steps of preparing a sample, of obtaining a hydrolyzed sample and of an extract.

### [Disclosure]

### [Technical Problem]

The present disclosure is directed to providing a method for simultaneous analysis of prohibited drugs and/or metabolites thereof in food by pretreatment and gas chromatography/mass spectrometry, which allows sensitive and fast detection of 90 or more prohibited drugs for a variety of food matrices.

### [Technical Solution]

The present disclosure provides a method for simultaneous analysis of prohibited drugs and/or metabolites thereof in food, which includes:
(a) a step of preparing a sample for analysis by extracting a food specimen with an alcohol;
(b) a step of obtaining a purified sample for analysis by purifying the sample for analysis with an ion-exchange resin;
(c) a step of obtaining a hydrolyzed sample for analysis by enzymatically treating the purified sample for analysis;
(d) a step of obtaining an extract of the sample for analysis by extracting the hydrolyzed sample for analysis with an organic solvent;
(e) a step of obtaining a derivatized sample for analysis by heat-treating the extract of the sample for analysis after adding a derivatizing agent; and
(f) a step of analyzing a plurality of prohibited drugs and/or metabolites thereof simultaneously by gas chromatography-mass spectrometry (GC-MS/MS) of the derivatized sample for analysis.

The prohibited drugs and/or the metabolite thereof may be one or more drug selected from an anabolic agent, a cannabinoid and an opiate.

In the step (a), the food specimen may be heat-treated with microwaves before the alcohol extraction if the food specimen is meat.

The heat treatment with microwaves may be performed with a power of 250-300 W.

In the step (a), the alcohol may be any one selected from methanol, ethanol, propanol and butanol.

In the step (a), the food specimen may be sonicated at 40-60 °C after the alcohol extraction.

In the step (b), the ion-exchange resin may be a nonpolar ion-exchange resin having a diameter of 0.1-0.2 mm.

If the food specimen is a food including a fatty substance, the step (b) may further include a freezing filtration step of freezing the fatty substance at -40 to -20 °C before the purification with the ion-exchange resin and then filtering the same.

In the step (c), a hydrolase used in the enzymatic treatment may be any one selected from β-glucuronidase and β-glucuronidase/arylsulfatase.

The hydrolysis may be performed at 50-60 °C.

In the step (c), the sample for analysis may be adjusted to weakly acidic pH of 6.0-6.8 before the hydrolysis.

In the step (c), the sample for analysis may be adjusted to basic pH of 9.0-10.0 after the hydrolysis.

In the step (d), the organic solvent may be any one selected from diisopropyl ether, methyl t-butyl ether (MTBE), ethyl t-butyl ether, methyl t-pentyl ether (TAME), ethyl t-pentyl ether, tetrahydrofuran (THF), 1,4-dioxane, ethylene glycol dimethyl ether or diethyl ether, diethylene glycol dimethyl ether and diethyl ether.

In the step (e), the derivatization may be trimethylsilylation.

The derivatizing agent may be a mixture including MSTFA (N-methyl-N-(trimethylsilyl)trifluoroacetamide), NH₄I and DTE (dithioerythritol).

In the derivatizing agent, the mixing ratio of MSTFA:NH₄I:DTE may be 500:3-5:1-3 (v/w/w).

The derivatization may be performed by heat-treating at 50-70 °C.

In the step (f), the analysis by gas chromatography-mass spectrometry (GC-MS/MS) may be performed by multiple reaction monitoring (MRM).

### [Advantageous Effects]

A method for simultaneous analysis of prohibited drugs and/or metabolites thereof in food of the present disclosure allows simultaneous analysis of 90 or more prohibited drugs included in meat including fatty substances, beverages including non-fatty substances, grains including non-fatty substances, oils, etc. by performing pretreatment including heat treatment using microwaves, delipidation by freezing filtration, purification with an ion-exchange resin, enzymatic degradation and derivatization and then performing simultaneous analysis by gas chromatography/mass spectrometry.

### [Brief Description of Drawings]

FIG. 1 is a flow chart illustrating a method for simultaneous analysis of prohibited drugs and/or metabolites thereof in food of the present disclosure.
FIG. 2 schematically shows an analysis module of the present disclosure depending on food matrices.
FIG. 3 shows photographic images illustrating a delipidation process.
FIG. 4 shows a photographic image illustrating a purification process using the ion-exchange resin PAD-1.
FIG. 5 shows the retention time and chromatographic data of prohibited drugs to be analyzed.
FIG. 6 shows a result of investigating prohibited drugs included in pork.

### [Best Mode]

Hereinafter, various aspects and exemplary embodiments of the present disclosure are described more specifically.

The exemplary embodiments of the present disclosure will be described in detail referring to the attached drawings so that those having ordinary knowledge in the art to which the present disclosure belongs can easily carry out the present disclosure.

However, the following description is not intended to limit the present disclosure to specific exemplary embodiments, and the detailed description of known related technologies may be omitted to avoid obscuring the present disclosure.

The terms used in the present specification are used only to describe specific exemplary embodiments, and are not intended to limit the present disclosure. Singular expressions include plural expressions unless the context clearly indicates otherwise. In the present specification, the terms such as "include", "have", etc. are intended to indicate the existence of a feature, number, step, operation, component or combinations thereof described in the specification, and it should be understood that the existence or possibility of addition of one or more features, numbers, steps, operations, components or combinations thereof is not precluded in advance.

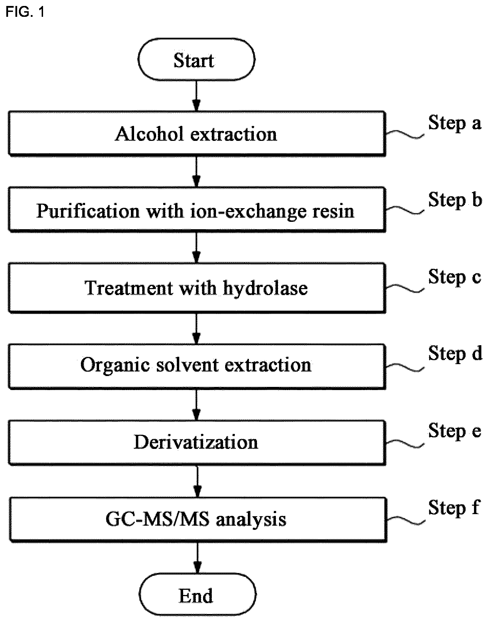

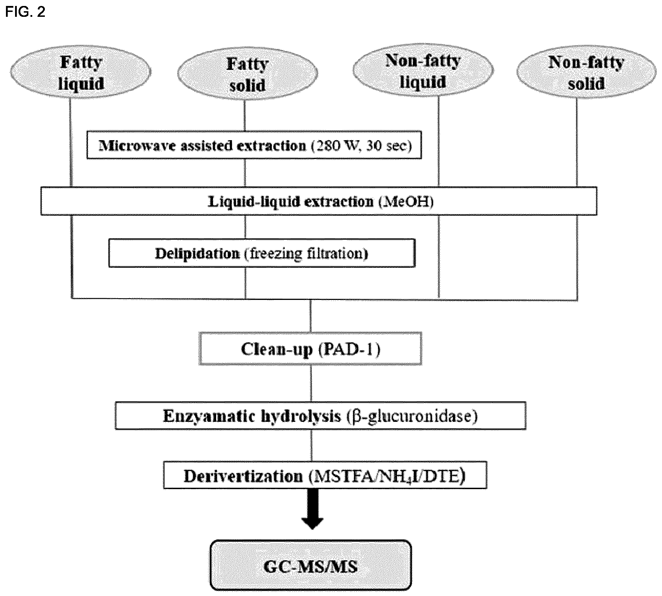

FIG. 1 is a flow chart illustrating a method for simultaneous analysis of prohibited drugs and/or metabolites thereof in food of the present disclosure, and FIG. 2 schematically shows an analysis module of the present disclosure depending on food matrices. Hereinafter, the method for simultaneous analysis of prohibited drugs and/or metabolites thereof of the present disclosure will be described referring to FIG. 1 and FIG. 2.

First, a sample for analysis is prepared by extracting a food specimen with an alcohol (step a).

The prohibited drugs and/or metabolites thereof may include one or more drug selected from an anabolic agent, a cannabinoid and an opiate.

Specifically, the prohibited drugs and/or metabolites thereof may include clenbuterol, nandrolone, testosterone, 19-NA (19-norandrosterone), 19-NE (19-noretiocholanolone), salbutamol, methyltestosterone, etc.

If the food specimen is meat such as pork, beef, etc., a step of heat-treating with microwaves may be performed before the alcohol extraction. However, for non-meat food, it is preferred to omit the step of heat-treating with microwaves.

The heat treatment with microwaves may be performed specifically with a power of 260-300 W, more specifically with a power of 270-290 W. Under such conditions, the heat treatment with microwaves may be performed specifically for 20-40 seconds, more specifically for 25-35 seconds.

An alcohol for the alcohol extraction may be specifically any one selected from methanol, ethanol, propanol and butanol, more specifically methanol.

After the alcohol extraction, sonication may be performed further specifically at 40-60 °C, more specifically at 45-55 °C. The sonication may be performed for 15-25 minutes.

Next, a purified sample for analysis is obtained by purifying the sample for analysis with an ion-exchange resin (step b).

Specifically, the ion-exchange resin may be a nonpolar ion-exchange resin having a diameter of 0.1-0.2 mm.

If the food specimen is a food including a fatty substance, a freezing filtration step of freezing the fatty substance at -40 to -20 °C and then filtering the same may be performed before the purification with the ion-exchange resin. More specifically, the fatty substance may be frozen at -35 to -25 °C and then filtered. Specifically, the fatty substance may be frozen for 3-5 minutes. Since the freezing point of fats is about 4 °C and the freezing point of methanol used as an extraction solvent is -98 °C, the fatty substance can be separated easily by the freezing treatment.

However, for vegetable beverages, grains, etc. that hardly include fatty substances, the freezing filtration step may be omitted.

After the delipidation and purification, concentration using a nitrogen concentrator and concentration using a rotary evaporator may be performed sequentially.

Then, a hydrolyzed sample for analysis is obtained by enzymatically treating the purified sample for analysis (step c).

A hydrolase used in the enzymatic treatment may be specifically β-glucuronidase, β-glucuronidase/arylsulfatase, etc., more specifically β-glucuronidase.

Hydrolysis may be performed at 50-60 °C, more specifically at 53-57 °C. The hydrolysis may be performed for 50-70 minutes, more specifically for 55-65 minutes.

Before the hydrolysis, the sample for analysis may be adjusted to weakly acidic pH of 6.0-6.8, more specifically to pH 6.5-6.7 by adding a phosphate buffer, etc.

Also, after the hydrolysis, the sample for analysis may be adjusted specifically to pH 9.0-10.0, more specifically to pH 9.5-9.7 using potassium carbonate, etc.

Next, an extract of the sample for analysis is obtained by extracting the hydrolyzed sample for analysis with an organic solvent (step d).

The organic solvent may be specifically any one selected from diisopropyl ether, methyl t-butyl ether (MTBE), ethyl t-butyl ether, methyl t-pentyl ether (TAME), ethyl t-pentyl ether, tetrahydrofuran (THF), 1,4-dioxane, ethylene glycol dimethyl ether or diethyl ether, diethylene glycol dimethyl ether and diethyl ether, more specifically methyl t-butyl ether (MTBE).

Then, a derivatized sample for analysis is obtained by heat-treating the extract of the sample for analysis after adding a derivatizing agent (step e).

The derivatization may be trimethylsilylation (TMS). The derivatized compound may be 19-norandrosterone di-TMS, androsterone di-TMS, boldenone di-TMS, morphine di-TMS, etc.

Specifically, the derivatizing agent may a mixture including MSTFA (N-methyl-N-(trimethylsilyl)trifluoroacetamide), NH₄I and DTE (dithioerythritol), and the mixing ratio of MSTFA:NH₄I:DTE in the derivatizing agent may be 500:3-5:1-3 (v/w/w).

The derivatization may be performed by heat-treating specifically at 50-70 °C, more specifically at 55-65 °C. The heat treatment may be performed specifically for 10-30 minutes, more specifically for 15-25 minutes. When the heat treatment temperature and time are outside the above-described ranges, derivatization may not be achieved sufficiently.

Finally, a plurality of prohibited drugs and/or metabolites thereof are analyzed simultaneously by gas chromatography-mass spectrometry (GC-MS/MS) of the derivatized sample for analysis (step Z

The gas chromatography-mass spectrometry (GC-MS/MS) may be performed by multiple reaction monitoring (MRM) to analyze a plurality of prohibited drugs simultaneously.

### [Test Example]

### Analysis method

### (1) Sample preparation

After preparing 1 g of ground pork (boar, barrow or sow) and adding 1 mL of water, heat treatment was performed for 30 seconds using microwaves (280 W). After adding an internal standard (ISTD) and 6 mL of methanol and then extracting ingredients, the extract was sonicated at 50 °C for 20 minutes. Then, a sample for analysis was prepared by centrifuging at 2000 g for 5 minutes.

### (2) Delipidation

After freezing the sample for analysis at -30 °C for 4 minutes and decanting on a filter paper, followed by evaporation with nitrogen for 40 minutes, the sample was concentrated with a rotary evaporator for 20 minutes.

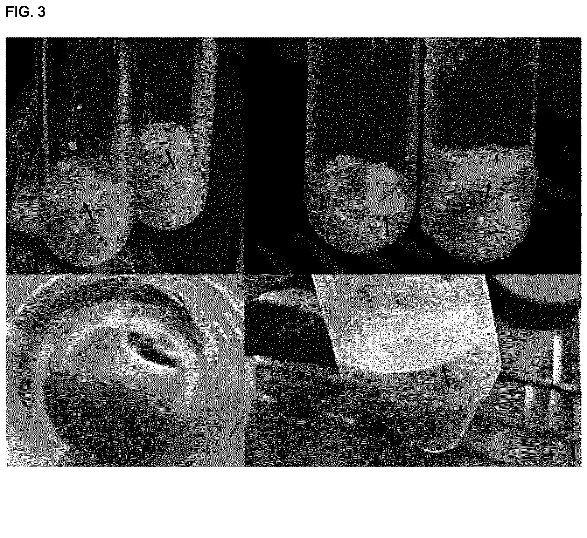

The process of delipidation by freezing is shown in the photographic images of FIG. 3.

### (3) Purification (clean-up) using ion-exchange resin (PDA-1)

After the delipidation process, the sample for analysis was diluted in 1 mL of water and applied to the nonpolar ion-exchange resin PDA-1 (Serdolit PAD-1, 0.1-0.2 mm (analytical grade), Serva). After filling 400 µL of the PDA-1 ion-exchange resin in a Pasteur pipette and washing with 2 mL of water, the sample was loaded onto the PDA-1 ion-exchange resin. After the loading, followed by washing with 2 mL of water, the sample was eluted with 4 mL of methanol. The diluted sample was evaporated with nitrogen for 40 minutes and then concentrated for 20 minutes using a rotary evaporator.

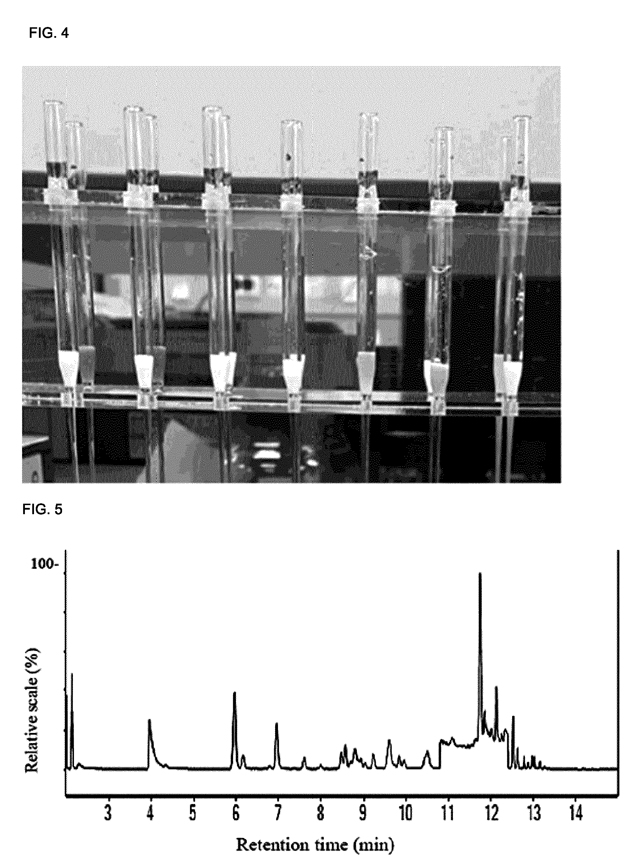

The purification process using the PDA-1 is shown in the photographic image of FIG. 4.

### (4) Enzymatic hydrolysis

After the purification process using the ion-exchange resin, the sample for analysis was adjusted to pH 6.7 using 1 mL of a phosphate buffer and then incubated at 55 °C for 60 minutes after adding 50 µL of β-glucuronidase. Then, the pH was adjusted to 9.6 by adding 0.7 mL of 5% potassium carbonate (K₂CO₃).

### (5) Extraction

After adding 5 mL of MTBE (methyl tertiary-butyl ether) to the enzymatically hydrolyzed sample for analysis and shaking for 10 minutes, the organic layer was separated by centrifuging at 2000 g for 5 minutes. Then, after concentrating with nitrogen for 15 minutes, the sample was dried for 30 minutes in a desiccator.

### (6) Derivatization

After adding 50 µL of MSTFA (N-methyl-N-(trimethylsilyl)trifluoroacetamide)/NH₄I/DTE (dithioerythritol) (v/w/w. 500:4:2) to the extracted sample for analysis and heat-treating at 60 °C for 20 minutes for derivatization, the sample was transferred to a vial of an autosampler.

### (7) GC-MS/MS analysis

The pretreated sample was analyzed by GC-MS/MS. An Agilent 7890B gas chromatography system equipped with a triple-quadrupole Agilent 7010 mass spectrometer was used. The GC, oven and MS conditions for the analysis are summarized in Table 1.

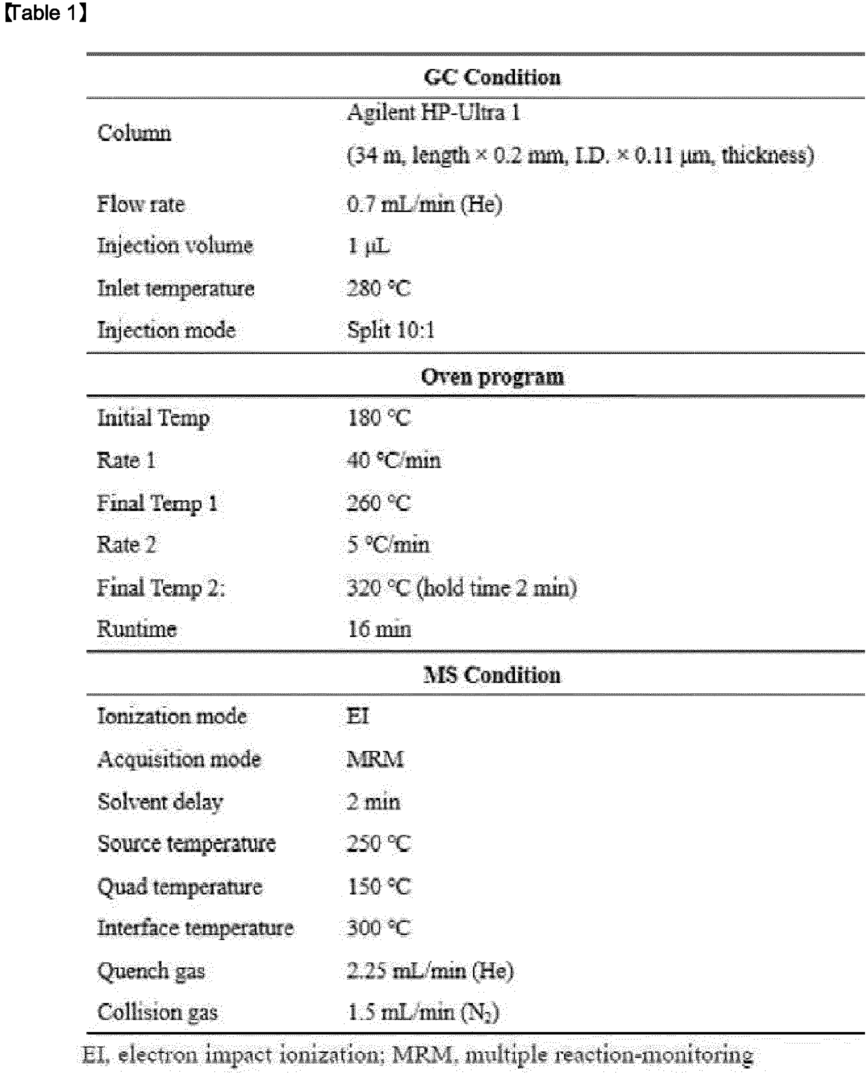

The materials were detected in an MRM (multiple reaction monitoring) analysis mode using the mass of precursor ions and product ions, and the mass and optimum collision energy of each product ion were determined by comparing with the internal standard.

### (8) Validity evaluation and quantitative evaluation

- Accuracy: It refers to the difference between a true value and a measurement result. It is calculated by comparing the analysis results obtained by adding a standard of at least three concentrations to a negative sample.
- Limit of detection: It refers to the lowest concentration detectable with a given instrument and can be determined in many ways. When the signal-to-noise (S/N) ratio is used, the limit of detection is generally determined as the concentration at which S/N is 3.

### Analysis result

### (1) Analytes and chromatograms

The mass of precursor ions and product ions was determined by loading the derivatized sample directly into a mass spectrometer and applying optimum collision energy (CE). Prohibited drugs are summarized in Table 2, and the measurement results for the prohibited drug are summarized in Table 3.

**[Table 2]**

| Name | Name | Name | Name | Name |
|---|---|---|---|---|
| Salbutamol | Calusterone-M | Zeranol | Norethisterone-M | Pregnanediol |
| Carphedone | Oxandrolone-M | Norethandrolone | 6b-OH-bromantan | Norboletone-M beta |
| Clenbuterol | Oxandrolone-LTM1 | Formestane | 1-Androsterone | 6-OXO |
| Letrozole-M | Bolasterone-M | Formebolone-M2 | | Oxabolone |
| Zilpaterol | Methyl-1-testosterone | Norclostebol | Fluoxymesterone-M2 | 6a-OH-androstenedione |
| d4-19-NA | Norethandrolone-M1 | Zeranol-M | 3b-OH-tibolone | 19-NT |
| 19-NA | Clostebol-M1 | Fluoxymesterone-M1 | 1-Testosterone | Epitrenbolone |
| Ethylestrenol | Metenolone | Norethandrolone-M2 | Mesterolone-M1 | Boldione |
| mono-TMS-An | Methylnortesoterone | 4-OH-testosterone | 1-Androstenediol | Mesterolone-M2 |
| Boldenone-M | Mestanolone | 4-OH-cyclofenil | Methyltestosterone-M1 | Tamoxifen-M |
| Methandienone-M1 | Mibolerone | 6b-OH-methandienone | Methyltestosterone-M2 | Fluoxymesterone-M3 |
| 19-NE | Methylstenebolone | a-Lenol | Androstenedione | 3'-OH-prostanozol |
| Morphine | DHCMT-M3 | Norboletone | 5-Androstendione | 4a/b-OH-prostanozol |
| 1-Testosterone-M | Methyldienolone | Fluoxymesterone | Methandienone-M9 | 3'-OH-stanozolol |
| Drostanolone-M | MT | DHCMT | Methasterone | 4b-OH-stanozolol |
| Bolandiol | Norboletone-M alpha | Oxymesterone | Calusterone | Stenebolone |
| 1-Androstenedione | THC-COOH | 2a-OH-methylethisterone | Ethisterone | 3a-OH-tibolone |
| Metenolone-M | Bolasterone | Methylclostebol | Oxandrolone | d3-Testosterone |
| Testosterone | Methasterone-M | Boldenone | Oxabolone-M | Mibolerone-M |

**[Table 3]**

| **Compounds** | **RT (min.)** | **Precursor ion (m/z)** | **Product ion (m/z)** | **CE (v)** |
|---|---|---|---|---|
| 1-Androstenediol | 9.07 | 434 | 405 | 10 |
| | | 434 | 195 | 20 |
| | | 434 | 377 | 10 |
| 1-Androstenedione | 8.61 | 415 | 221 | 20 |
| | | 194 | 105 | 20 |
| 1-Testosterone | 8.92 | 432 | 206 | 10 |
| | | 432 | 194 | 10 |
| 1-Testosterone-M | 7.76 | 432 | 290 | 10 |
| | | 432 | 403 | 10 |
| | | 432 | 275 | 20 |
| 4-OH-testosterone | 12.64 | 520 | 431 | 20 |
| | | 520 | 225 | 25 |
| Bolandiol | 8.16 | 420 | 240 | 5 |
| | | 420 | 391 | 10 |
| Bolasterone | 11.96 | 315 | 169 | 20 |
| | | 355 | 169 | 30 |
| Bolasterone-M | 10.6 | 374 | 269 | 10 |
| | | 374 | 227 | 10 |
| Calusterone | 12.6 | 445 | 355 | 10 |
| | | 445 | 225 | 15 |
| Calusterone-M | 10.11 | 269 | 159 | 20 |
| | | 269 | 213 | 10 |
| Clostebol-M | 10.62 | 466 | 431 | 10 |
| | | 466 | 169 | 30 |
| Drostanolone-M | 8.15 | 448 | 343 | 15 |
| | | 448 | 169 | 30 |
| Ethylestrenol | 6.18 | 270 | 241 | 10 |
| | | 270 | 185 | 20 |
| Fluoxymesterone | 12.98 | 552 | 462 | 10 |
| | | 552 | 407 | 15 |
| Danazol-M | 13.11 | 558 | 193 | 40 |
| | | 558 | 403 | 20 |
| Methandienone-M2 | 12.78 | 571 | 337 | 20 |
| | | 517 | 429 | 20 |
| DHCMT-M3 | 11.7 | 379 | 343 | 5 |
| | | 379 | 253 | 15 |
| | | 379 | 225 | 15 |
| Norethisterone-M | 8.54 | 431 | 193 | 20 |
| | | 431 | 167 | 20 |
| | | 431 | 341 | 15 |
| Oxabolone-M | 11.05 | 504 | 219 | 35 |
| | | 504 | 234 | 20 |
| Oxandrolone-M | 10.16 | 308 | 176 | 5 |
| | | 324 | 189 | 20 |
| Fluoxymesterone-M 1 | 12.62 | 642 | 447 | 10 |
| | | 642 | 427 | 20 |
| | | 642 | 357 | 20 |
| Fluoxymesterone-M2 | 12.55 | 462 | 208 | 20 |
| | | 462 | 357 | 15 |
| Fluoxymesterone-M3 | 13.36 | 640 | 605 | 25 |
| | | 640 | 515 | 20 |
| | | 640 | 425 | 25 |
| Formebolone M2 | 12.55 | 534 | 389 | 20 |
| | | 534 | 301 | 20 |
| Mestanolone | 11.21 | 216 | 159 | 10 |
| | | 216 | 187 | 10 |
| Mesterolone-M1 | 9.03 | 433 | 343 | 5 |
| | | 433 | 253 | 10 |
| Mesterolone-M2 | 9.38 | 450 | 255 | 20 |
| | | 345 | 255 | 10 |
| Methandienone-M1 | 6.39 | 358 | 301 | 15 |
| | | 358 | 169 | 30 |
| Methandienone-M9 | 9.67 | 442 | 236 | 10 |
| | | 442 | 339 | 10 |
| Metenolone | 10.63 | 446 | 195 | 20 |
| | | 446 | 208 | 15 |
| Metenolone-M | 8.63 | 446 | 431 | 10 |
| | | 446 | 251 | 20 |
| Methasterone | 12 | 462 | 419 | 10 |
| | | 332 | 156 | 10 |
| Methasterone-M | 9.7 | 449 | 269 | 10 |
| | | 449 | 359 | 10 |
| Methylclostebol | 13.15 | 480 | 390 | 10 |
| | | 480 | 335 | 20 |
| Methyldienolone | 11.74 | 430 | 285 | 20 |
| | | 430 | 340 | 15 |
| | | 430 | 246 | 20 |
| Methyl-1-testosterone | 10.63 | 206 | 165 | 20 |
| | | 446 | 206 | 10 |
| Methylnortesoterone | 10.99 | 287 | 207 | 20 |
| | | 287 | 197 | 10 |
| Methylstenebolone | 11.64 | 370 | 220 | 10 |
| | | 370 | 245 | 20 |
| | | 370 | 231 | 30 |
| Methyltestosterone-M1 | 9.08 | 435 | 255 | 15 |
| | | 435 | 173 | 30 |
| Methyltestosterone-M2 | 9.17 | 270 | 213 | 10 |
| | | 270 | 199 | 20 |
| Mibolerone | 11.23 | 431 | 341 | 10 |
| | | 301 | 73 | 40 |
| Mibolerone-M | 9.75 | 360 | 255.2 | 15 |
| | | 360 | 213.1 | 15 |
| Norboletone | 12.98 | 460 | 301 | 10 |
| | | 301 | 143 | 30 |
| Norboletone-M alpha | 11.82 | 435 | 255 | 20 |
| | | 435 | 345 | 10 |
| Norboletone-M beta | 12.16 | 435 | 255 | 20 |
| | | 435 | 345 | 10 |
| | | 144 | 75 | 20 |
| Norclostebol | 12.6 | 452 | 321 | 20 |
| | | 452 | 417 | 5 |
| Norethandrolone | 12.53 | 446 | 287 | 20 |
| | | 446 | 300 | 20 |
| Norethandrolone-M1 | 10.64 | 421 | 241 | 15 |
| | | 331 | 241 | 10 |
| Norethandrolone-M2 | 12.63 | 421 | 331 | 5 |
| | | 421 | 241 | 15 |
| Oxabolone | 12.27 | 506 | 147 | 35 |
| | | 506 | 195 | 30 |
| Oxandrolone | 12.07 | 363 | 161 | 20 |
| | | 308 | 161 | 20 |
| | | 321 | 95 | 35 |
| Oxandrolone LTM1 | 10.15 | 273 | 213 | 10 |
| | | 273 | 161 | 20 |
| Oxymesterone | 13.02 | 534 | 301 | 40 |
| | | 389 | 169 | 30 |
| | | 389 | 257 | 20 |
| 3'-OH-prostanozolol | 13.45 | 544 | 254 | 30 |
| | | 529 | 347 | 45 |
| 4α/β-H-prostanozol | 13.63 | 544 | 112 | 40 |
| | | 544 | 211 | 30 |
| 3'-OH-stanozolol | 13.75 | 560 | 520 | 10 |
| | | 560 | 254 | 15 |
| | | 545 | 387 | 50 |
| 4β-OH-stanozolol | 13.76 | 545 | 381 | 40 |
| | | 380 | 213 | 15 |
| Stenbolone | 9.79 | 446 | 208 | 15 |
| | | 446 | 220 | 15 |
| Androstenedione | 9.65 | 430 | 415 | 20 |
| | | 430 | 209 | 20 |
| Boldenone | 9.73 | 430 | 206 | 15 |
| | | 206 | 165 | 15 |
| | | 325 | 229 | 20 |
| Boldenone-M | 6.38 | 194 | 163 | 20 |
| | | 194 | 149 | 20 |
| | | 432 | 194 | 10 |
| | | 432 | 179 | 40 |
| Boldione | 9.38 | 428 | 206 | 15 |
| | | 413 | 229 | 20 |
| Testoterone | 9.98 | 432 | 209 | 20 |
| 5-Androstendione | 9.65 | 430 | 415 | 20 |
| | | 430 | 209 | 20 |
| 19-Norandrosterone | 6.16 | 405 | 315 | 5 |
| 19-Noretiocholanolone | 7 | 405 | 225 | 15 |
| | | 405 | 315 | 5 |
| | | 405 | 225 | 10 |
| Nandrolone | 9.13 | 418 | 194 | 18 |
| Clenbuterol | 2.84 | 335 | 300 | 10 |
| | | 337 | 302 | 10 |
| 3α-OH-tibolone | 9.85 | 443 | 193 | 30 |
| | | 443 | 167 | 25 |
| 3β-OH-tibolone | 8.92 | 443 | 193 | 30 |
| | | 443 | 167 | 25 |
| 4-tibolone | 11.49 | 441 | 247 | 15 |
| | | 441 | 351 | 10 |
| Zilpaterol | 4.52 | 308 | 218 | 15 |
| | | 405 | 308 | 5 |
| | | 308 | 203 | 30 |
| Zeranol | 12.52 | 433 | 295 | 25 |
| | | 433 | 389 | 10 |
| 4-OH-cyclofenil | 12.78 | 422 | 367 | 20 |
| | | 422 | 343 | 25 |
| 6-OH-bromantan | 8.73 | 395 | 133 | 15 |
| | | 393 | 171 | 20 |
| 6-OXO | 12.19 | 516 | 411 | 15 |
| | | 516 | 205 | 20 |
| 6-OXO-M | 12.35 | 518 | 319 | 30 |
| | | 518 | 413 | 20 |
| Carphedone | 2.47 | 272 | 104 | 20 |
| | | 272 | 257 | 10 |
| Formestane | 12.54 | 518 | 221 | 30 |
| | | 518 | 429 | 15 |
| Letrozole-M | 4.07 | 291 | 160 | 10 |
| | | 291 | 217 | 20 |
| Morphine | 7.14 | 429 | 287 | 20 |
| | | 429 | 414 | 15 |
| Salbutamol | 2.43 | 369 | 191 | 20 |
| | | 369 | 295 | 15 |
| Tamoxifen-M | 13.22 | 489 | 72 | 10 |
| | | 489 | 58 | 25 |
| THC-COOH | 11.89 | 473 | 355 | 30 |
| | | 371 | 315 | 15 |
| α-Zearalenol | 12.78 | 446 | 317 | 25 |
| | | 536 | 317 | 40 |
| β-Zearaleno | 12.87 | 446 | 317 | 25 |
| | | 536 | 317 | 40 |
| mono-TMS-an | 6.32 | 347 | 271 | 10 |
| Methyltestosterone | 11.81 | 446 | 301 | 20 |
| d₃-Testosterone | 9.96 | 435 | 209 | 20 |
| d₄-19-NA | 9.1 | 409 | 319 | 5 |

GC-MS/MS analysis was performed by adding the data of Table 3 to an MRM (multiple reaction monitoring) mode. The retention time and chromatographic data are shown in FIG. 5. All the analytes were detected within 2-14 minutes, independently without interference between each other.

### (2) Verification of analysis method

Recovery rate was determined by recovery test and limit of detection (LOD) was determined using a blank matrix. As a result, the limit of detection was measured as 0.005-20 µg/kg and the recovery rate was in a range from 80.42% to 122.58%. The result of verifying the analysis method is summarized in Table 4.

**[Table 4]**

| Name | Spiked Conc. (µg/kg) | Recovery (%) | LOD (µg/kg) |
|---|---|---|---|
| Salbutamol | 200 | 82.15 | 0.5 |
| Carphedone | 50 | 88.35 | 2.5 |
| Clenbuterol | 1 | 82.85 | 0.005 |
| Letrozole-M | 10 | 95.54 | 0.25 |
| Zilpaterol | 2 | 96.59 | 0.2 |
| 19-NA | 15 | 82.53 | 0.0375 |
| Ethylestrenol | 5 | 83.86 | 0.125 |
| Boldenone-M | 5 | 81.06 | 0.25 |
| Methandienone-M1 | 2 | 81.85 | 0.4 |
| 19-NE | 2 | 80.42 | 0.4 |
| Morphine | 200 | 92.89 | 0.5 |
| 1-Testosterone-M | 5 | 96.82 | 0.125 |
| Drostanolone-M | 5 | 94.68 | 0.5 |
| Bolandiol | 5 | 91.66 | 10 |
| 1-Androstenedione | 5 | 95.90 | 0.25 |
| Metenolone-M | 5 | 100.88 | 0.25 |
| Norethisterone-M | 5 | 96.67 | 0.125 |
| 6b-OH-bromantan | 10 | 102.47 | 2.5 |
| 1-Androsterone | 5 | 89.70 | 0.5 |
| Fluoxymesterone-M2 | 5 | 102.97 | 0.125 |
| 3b-OH-tibolone | 2 | 86.85 | 0.1 |
| 1-Testosterone | 5 | 113.98 | 1 |
| Mesterolone-M1 | 10 | 95.88 | 1 |
| 1-Androstenediol | 5 | 112.05 | 1 |
| Methyltestosterone-M 1 | 2 | 84.89 | 0.5 |
| Methyltestosterone-M2 | 2 | 91.61 | 1 |
| 19-NT | 1 | 82.49 | 1 |
| Epitrenbolone | 5 | 120.00 | 5 |
| Boldione | 5 | 87.81 | 2.5 |
| Mesterolone-M2 | 5 | 92.21 | 1 |
| Methandienone-M9 | 5 | 104.22 | 5 |
| Methasterone-M | 5 | 106.71 | 0.25 |
| Boldenone | 5 | 97.41 | 1 |
| Stenebolone | 5 | 92.99 | 0.25 |
| 3a-OH-tibolone | 2 | 95.01 | 0.1 |
| Testosterone | 1 | 99.62 | 0.1 |
| Calusterone-M | 5 | 91.82 | 1 |
| Bolasterone-M | 5 | 99.79 | 0.5 |
| Methyl-1-testosterone | 5 | 102.53 | 5 |
| Norethandrolone-M1 | 5 | 92.14 | 0.25 |
| Clostebol-M 1 | 5 | 92.67 | 1.25 |
| Metenolone | 5 | 100.06 | 1.25 |
| Methylnortesoterone | 5 | 106.02 | 2.5 |
| Mestanolone | 5 | 87.40 | 1 |
| Mibolerone | 5 | 91.15 | 5 |
| Delta4-tibolone | 2 | 89.57 | 1 |
| Methylstenebolone | 5 | 122.58 | 5 |
| DHCMT-M3 | 2 | 85.22 | 0.4 |
| Methyldienolone | 5 | 105.76 | 5 |
| Norboletone-M alpha | 5 | 98.15 | 0.125 |
| THC-COOH | 50 | 81.14 | 0.125 |
| Bolasterone | 5 | 90.01 | 0.5 |
| Methasterone | 5 | 110.59 | 0.5 |
| Calusterone | 5 | 115.82 | 2.5 |
| Ethisterone | 5 | 109.32 | 1 |
| Norboletone-M beta | 5 | 93.23 | 1 |
| 6-OXO | 10 | 104.99 | 2 |
| Oxabolone | 5 | 111.65 | 1 |
| 6-OXO-M | 5 | 114.36 | 10 |
| Zeranol | 2 | 86.27 | 0.4 |
| Norethandrolone | 5 | 100.72 | 1 |
| Formestane | 50 | 112.81 | 1.25 |
| Norclostebol | 5 | 109.34 | 10 |
| Zeranol-M | 2 | 108.29 | 0.4 |
| Fluoxymesterone-M 1 | 20 | 95.42 | 20 |
| Norethandrolone-M2 | 5 | 114.17 | 0.125 |
| 4-OH-testosterone | 5 | 111.6 | 1 |
| 4-OH-cyclofenil | 10 | 102.71 | 0.25 |
| Methandienone-M2 | 2 | 93.56 | 2 |
| a-Lenol | 5 | 117.33 | 1 |
| Norboletone | 5 | 112.63 | 1 |
| Fluoxymesterone | 5 | 120.06 | 1.25 |
| DHCMT | 2 | 102.90 | 1 |
| Oxymesterone | 5 | 108.01 | 2.5 |
| Danazol-M2 | 5 | 94.75 | 1 |
| Methylclostebol | 5 | 91.73 | 1 |
| Tamoxifen-M | 10 | 112.43 | 5 |
| Fluoxymesterone-M3 | 5 | 89.14 | 10 |
| b-lenol | 0.5 | 112.36 | 0.1 |

### (3) Result of fact finding

Fact finding was performed for commercially available samples. The samples used for the fact finding are summarized in Table 5.

**[Table 5]**

| Food matrices | Samples |
|---|---|
| Non-fatty liquids | Fig juice and oat milk |
| Non-fatty solids | Wheat, maize, barley, oats, poppy seed, fig, maize and hempseed |
| Fatty liquids | Hempseed oil, and lard oil |
| Fatty solids | Pork (sow and boar), chicken, beef and pig testicles |

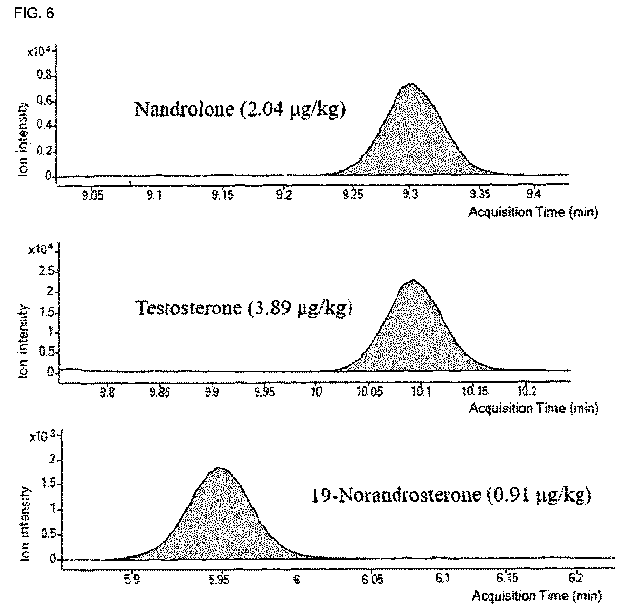

The result of analyzing prohibited drugs for the meat of boar (testicles), barrow and sow among the samples for fact finding is summarized in Table 6 and FIG. 6.

**[Table 6]**

| Pork samples | Concentration (µg/kg) | |
|---|---|---|
| Boar | Nandrolone | 2.04 |
| | Testosterone | 3.89 |
| Barrow | 19-Norandrosterone | 0.56 |
| | Testosterone | 0.15 |
| Sow | 19-Norandrosterone | 0.31-1.4 (3) |
| | Testosterone | 0.15 |
| | 19-Noretiocholanolone | 0.41 |

As a result of analyzing the three pork samples, nandrolone (2.04 µg/kg), 19-norandrosterone (0.31-1.4 µg/kg), testosterone (0.15-3.89 µg/kg) and 19-noretiochloanolone (0.41 µg/kg) were detected.

## Claims

1. A method for simultaneous analysis of prohibited drugs and/or metabolites thereof in food, comprising:
(a) a step of preparing a sample for analysis by extracting a food specimen with an alcohol;
(b) a step of obtaining a purified sample for analysis by purifying the sample for analysis with an ion-exchange resin;
(c) a step of obtaining a hydrolyzed sample for analysis by enzymatically treating the purified sample for analysis;
(d) a step of obtaining an extract of the sample for analysis by extracting the hydrolyzed sample for analysis with an organic solvent;
(e) a step of obtaining a derivatized sample for analysis by heat-treating the extract of the sample for analysis after adding a derivatizing agent; and
(f) a step of analyzing a plurality of prohibited drugs and/or metabolites thereof simultaneously by gas chromatography-mass spectrometry (GC-MS/MS) of the derivatized sample for analysis.

2. The method for simultaneous analysis of prohibited drugs and/or metabolites thereof in food according to claim 1, wherein the prohibited drugs and/or the metabolite thereof is one or more drug selected from an anabolic agent, a cannabinoid and an opiate.

3. The method for simultaneous analysis of prohibited drugs and/or metabolites thereof in food according to claim 1 or 2, wherein, in the step (a), the food specimen is heat-treated with microwaves before the alcohol extraction if the food specimen is meat.

4. The method for simultaneous analysis of prohibited drugs and/or metabolites thereof in food according to claim 3, wherein the heat treatment with microwaves is performed with a power of 250-300 W.

5. The method for simultaneous analysis of prohibited drugs and/or metabolites thereof in food according to one of claims 1 to 4, wherein, in the step (a), the food specimen is sonicated at 40-60 °C after the alcohol extraction.

6. The method for simultaneous analysis of prohibited drugs and/or metabolites thereof in food according to one of claims 1 to 5, wherein the ion-exchange resin is a nonpolar ion-exchange resin having a diameter of 0.1-0.2 mm.

7. The method for simultaneous analysis of prohibited drugs and/or metabolites thereof in food according to one of claims 1 to 6, wherein, if the food specimen is a food including a fatty substance, the step (b) further comprises a freezing filtration step of freezing the fatty substance at -40 to -20 °C before the purification with the ion-exchange resin and then filtering the same.

8. The method for simultaneous analysis of prohibited drugs and/or metabolites thereof in food according to one of claims 1 to 7, wherein, in the step (c), a hydrolase used in the enzymatic treatment is any one selected from β-glucuronidase and β-glucuronidase/arylsulfatase.

9. The method for simultaneous analysis of prohibited drugs and/or metabolites thereof in food according to one of claims 1 to 8, wherein, in the step (c), the sample for analysis is adjusted to weakly acidic pH of 6.0-6.8 before the hydrolysis.

10. The method for simultaneous analysis of prohibited drugs and/or metabolites thereof in food according to one of claims 1 to 9, wherein, in the step (c), the sample for analysis is adjusted to basic pH of 9.0-10.0 after the hydrolysis.

11. The method for simultaneous analysis of prohibited drugs and/or metabolites thereof in food according to one of claims 1 to 10, wherein, in the step (d), the organic solvent is any one selected from diisopropyl ether, methyl t-butyl ether (MTBE), ethyl t-butyl ether, methyl t-pentyl ether (TAME), ethyl t-pentyl ether, tetrahydrofuran (THF), 1,4-dioxane, ethylene glycol dimethyl ether or diethyl ether, diethylene glycol dimethyl ether and diethyl ether.

12. The method for simultaneous analysis of prohibited drugs and/or metabolites thereof in food according to one of claims 1 to 11, wherein, the step (e), the derivatization is trimethylsilylation.

13. The method for simultaneous analysis of prohibited drugs and/or metabolites thereof in food according to one of claims 1 to 12, wherein the derivatizing agent is a mixture comprising MSTFA **(N-**methyl-N-(trimethylsilyl)trifluoroacetamide), NH₄I and DTE (dithioerythritol).

14. The method for simultaneous analysis of prohibited drugs and/or metabolites thereof in food according to claim 13, wherein the mixing ratio of MSTFA:NH₄I:DTE is 500:3-5:1-3 (v/w/w).

15. The method for simultaneous analysis of prohibited drugs and/or metabolites thereof in food according to one of claims 1 to 14, wherein, in the step (f), the analysis by gas chromatography-mass spectrometry (GC-MS/MS) is performed by multiple reaction monitoring (MRM).

## Patentansprüche

1. Verfahren zur gleichzeitigen Analyse verbotener Substanzen und/oder deren Metaboliten in Nahrungsmitteln, umfassend folgende Verfahrensschritte:
(a) Vorbereiten einer zu analysierenden Probe durch Extrahieren einer Nahrungsmittelprobe mit einem Alkohol;
(b) Erhalten einer aufgereinigten zu analysierenden Probe durch Aufreinigung der zu analysierenden Probe mit einem Ionenaustauscherharz;
(c) Erhalten einer hydrolysierten zu analysierenden Probe durch enzymatische Behandlung der aufgereinigten zu analysierenden Probe;
(d) Erhalten eines Extrakts der zu analysierenden Probe durch Extrahieren der hydrolysierten zu analysierenden Probe mit einem organischen Lösungsmittel;
(e) Erhalten einer derivatisierten zu analysierenden Probe durch thermische Behandlung des Extrakts der zu analysierenden Probe nach Zugabe eines Derivatisierungsmittels; und
(f) gleichzeitiges Analysieren einer Vielzahl verbotener Substanzen und/oder deren Metaboliten durch Gaschromatografie-Massenspektrometrie (GC-MS/MS) der derivatisierten zu analysierenden Probe.

2. Verfahren zur gleichzeitigen Analyse verbotener Substanzen und/oder deren Metaboliten in Nahrungsmitteln nach Anspruch 1, wobei es sich bei den verbotenen Medikamenten und/oder deren Metaboliten um ein Anabolikum, ein Cannabinoid und/oder ein Opiat handelt.

3. Verfahren zur gleichzeitigen Analyse verbotener Substanzen und/oder deren Metaboliten in Nahrungsmitteln nach Anspruch 1 oder 2, wobei die Nahrungsmittelprobe in Schritt (a) vor der Alkoholextraktion mit Mikrowellen thermisch behandelt wird, wenn es sich bei der Nahrungsmittelprobe um Fleisch handelt.

4. Verfahren zur gleichzeitigen Analyse verbotener Substanzen und/oder deren Metaboliten in Nahrungsmitteln nach Anspruch 3, wobei die thermische Behandlung mit Mikrowellen bei einer Leistung von 250 bis 300 W erfolgt.

5. Verfahren zur gleichzeitigen Analyse verbotener Substanzen und/oder deren Metaboliten in Nahrungsmitteln nach einem der Ansprüche 1 bis 4, wobei die Nahrungsmittelprobe in Schritt (a) nach der Alkoholextraktion bei 40 bis 60 °C im Ultraschallbad behandelt wird.

6. Verfahren zur gleichzeitigen Analyse verbotener Substanzen und/oder deren Metaboliten in Nahrungsmitteln nach einem der Ansprüche 1 bis 5, wobei es sich bei dem Ionenaustauscherharz um ein unpolares Ionenaustauscherharz mit einem Durchmesser von 0,1 bis 0,2 mm handelt.

7. Verfahren zur gleichzeitigen Analyse verbotener Substanzen und/oder deren Metaboliten in Nahrungsmitteln nach einem der Ansprüche 1 bis 6, wobei, wenn es sich bei der Nahrungsmittelprobe um ein eine fettige Substanz enthaltendes Nahrungsmittel handelt, der Schritt (b) zusätzlich einen Gefrierfiltrationsschritt umfasst, wobei die fettige Substanz vor der Aufreinigung mit dem Ionenaustauscherharz auf -40 bis -20 °C eingefroren und anschließend abfiltriert wird.

8. Verfahren zur gleichzeitigen Analyse verbotener Substanzen und/oder deren Metaboliten in Nahrungsmitteln nach einem der Ansprüche 1 bis 7, wobei es sich bei einer bei der enzymatischen Behandlung in Schritt (c) verwendeten Hydrolase um eine aus β-Glucuronidase und β-Glucuronidase/Arylsulfatase ausgewählte Hydrolase handelt.

9. Verfahren zur gleichzeitigen Analyse verbotener Substanzen und/oder deren Metaboliten in Nahrungsmitteln nach einem der Ansprüche 1 bis 8, wobei die zu analysierende Probe in Schritt (c) vor der Hydrolyse auf einen schwach sauren pH-Wert von 6,0 bis 6,8 eingestellt wird.

10. Verfahren zur gleichzeitigen Analyse verbotener Substanzen und/oder deren Metaboliten in Nahrungsmitteln nach einem der Ansprüche 1 bis 9, wobei die zu analysierende Probe in Schritt (c) nach der Hydrolyse auf einen basischen pH-Wert von 9,0 bis 10,0 eingestellt wird.

11. Verfahren zur gleichzeitigen Analyse verbotener Substanzen und/oder deren Metaboliten in Nahrungsmitteln nach einem der Ansprüche 1 bis 10, wobei es sich bei dem organischen Lösungsmittel in Schritt (d) um ein organisches Lösungsmittel, ausgewählt aus Diisopropylether, Methyl-t-butylether (MTBE), Ethyl-t-butylether, Methyl-t-pentylether (TAME), Ethyl-t-pentylether, Tetrahydrofuran (THF), 1,4-Dioxan, Ethylenglycoldimethylether oder -diethylether, Diethylenglycoldimethylether und Diethylether handelt.

12. Verfahren zur gleichzeitigen Analyse verbotener Substanzen und/oder deren Metaboliten in Nahrungsmitteln nach einem der Ansprüche 1 bis 11, wobei es sich bei der Derivatisierung in Schritt (e) um eine Trimethylsilylierung handelt.

13. Verfahren zur gleichzeitigen Analyse verbotener Substanzen und/oder deren Metaboliten in Nahrungsmitteln nach einem der Ansprüche 1 bis 12, wobei es sich bei dem Derivatisierungsmittel um ein Gemisch umfassend MSTFA (N-Methyl-N-(trimethylsilyl)trifluoracetamid), NH₄I und DTE (Dithioerythritol) handelt.

14. Verfahren zur gleichzeitigen Analyse verbotener Substanzen und/oder deren Metaboliten in Nahrungsmitteln nach Anspruch 13, wobei das Mischungsverhältnis von MSTFA : NH₄I : DTE 500 : 3 bis 5 : 1 bis 3 (Vol./Gew./Gew.) beträgt.

15. Verfahren zur gleichzeitigen Analyse verbotener Substanzen und/oder deren Metaboliten in Nahrungsmitteln nach einem der Ansprüche 1 bis 14, wobei die Analyse durch Gaschromatografie-Massenspektrometrie (GC-MS/MS) in Schritt (f) nach einem Multiple-Reaction-Monitoring-Verfahren (MRM-Verfahren) erfolgt.

## Revendications

1. Procédé d'analyse simultanée de substances interdits et/ou de leurs métabolites dans des aliments, le procédé comprenant :
(a) une étape consistant à préparer un échantillon à analyser en extrayant un spécimen d'aliment avec de l'alcool ;
(b) une étape consistant à obtenir un échantillon purifié à analyser en purifiant l'échantillon à analyser avec une résine échangeuse d'ions ;
(c) une étape consistant à obtenir un échantillon hydrolysé à analyser en traitant de manière enzymatique l'échantillon purifié à analyser ;
(d) une étape consistant à obtenir un extrait de l'échantillon à analyser en extrayant l'échantillon hydrolysé à analyser avec un solvant organique ;
(e) une étape consistant à obtenir un échantillon dérivé à analyser en traitant thermiquement l'extrait de l'échantillon à analyser après avoir ajouté un agent de dérivation ; et
(f) une étape consistant à analyser simultanément une pluralité de substances interdits et/ou de leurs métabolites par chromatographie en phase gazeuse-spectrométrie de masse (GC-MS/MS) de l'échantillon dérivé à analyser.

2. Procédé d'analyse simultanée de substances interdits et/ou de leurs métabolites dans des aliments selon la revendication 1, dans lequel les substances interdits et/ou leur métabolite est un ou plusieurs sélectionné(s) parmi un agent anabolique, un cannabinoïde et un opioïde.

3. Procédé d'analyse simultanée de substances interdits et/ou de leurs métabolites dans des aliments selon la revendication 1 ou 2, dans lequel, dans l'étape (a), le spécimen d'aliment est traité thermiquement avec des micro-ondes avant l'extraction à l'alcool si le spécimen d'aliment est de la viande.

4. Procédé d'analyse simultanée de substances interdits et/ou de leurs métabolites dans des aliments selon la revendication 3, dans lequel le traitement thermique avec des micro-ondes est effectué à une puissance de 250 à 300 W.

5. Procédé d'analyse simultanée de substances interdits et/ou de leurs métabolites dans des aliments selon l'une quelconque des revendication 1 à 4, dans lequel, dans l'étape (a), le spécimen d'aliment est soniqué à de 40 à 60 °C après l'extraction à l'alcool.

6. Procédé d'analyse simultanée de substances interdits et/ou de leurs métabolites dans des aliments selon l'une quelconque des revendication 1 à 5, dans lequel la résine échangeuse d'ions est une résine échangeuse d'ions non polaire ayant un diamètre de 0,1 à 0,2 mm.

7. Procédé d'analyse simultanée de substances interdits et/ou de leurs métabolites dans des aliments selon l'une quelconque des revendication 1 à 6, dans lequel, si le spécimen d'aliment est un aliment contenant une substance grasse, l'étape (b) comprend en outre une étape de congélation/filtration consistant à congeler la substance grasse à de -40 à -20 °C avant la purification avec la résine échangeuse d'ions et puis filtrer la substance grasse.

8. Procédé d'analyse simultanée de substances interdits et/ou de leurs métabolites dans des aliments selon l'une quelconque des revendication 1 à 7, dans lequel, dans l'étape (c), une hydrolase utilisée dans le traitement enzymatique est une hydrolase sélectionnée parmi la β-glucuronidase et la β-glucuronidase/arylsulfatase.

9. Procédé d'analyse simultanée de substances interdits et/ou de leurs métabolites dans des aliments selon l'une quelconque des revendication 1 à 8, dans lequel, dans l'étape (c), l'échantillon à analyser est ajusté à un pH faiblement acide de 6,0 à 6,8 avant l'hydrolyse.

10. Procédé d'analyse simultanée de substances interdits et/ou de leurs métabolites dans des aliments selon l'une quelconque des revendication 1 à 9, dans lequel, dans l'étape (c), l'échantillon à analyser est ajusté à un pH basique de 9,0 à 10,0 après l'hydrolyse.

11. Procédé d'analyse simultanée de substances interdits et/ou de leurs métabolites dans des aliments selon l'une quelconque des revendication 1 à 10, dans lequel, dans l'étape (d), le solvant organique est un solvant organique sélectionné parmi l'éther, le méthyl-t-butyl-éther (MTBE), l'éthyl-t-butyl-éther, le méthyl-t-pentyléther (TAME), l'éthyl-t-pentyl-éther, le tétrahydrofurane (THF), le 1,4-dioxane, l'éthylène glycol diméthyl éther ou diéthyl éther, le diéthylène glycol diméthyl éther et le diéthyl éther.

12. Procédé d'analyse simultanée de substances interdits et/ou de leurs métabolites dans des aliments selon l'une quelconque des revendication 1 à 11, dans lequel, dans l'étape (e), la dérivation est une triméthylsilylation.

13. Procédé d'analyse simultanée de substances interdits et/ou de leurs métabolites dans des aliments selon l'une quelconque des revendication 1 à 12, dans lequel l'agent de dérivation est un mélange comprenant du MSTFA (N-méthyl-N-(triméthylsilyl)trifluoroacétamide), du NH₄I et du DTE (dithioérythritol).

14. Procédé d'analyse simultanée de substances interdits et/ou de leurs métabolites dans des aliments selon la revendication 13, dans lequel le rapport de mélange de MSTFA : NH₄I : DTE est de 500 : 3 à 5 : 1 à 3 (volume/poids/poids).

15. Procédé d'analyse simultanée de substances interdits et/ou de leurs métabolites dans des aliments selon l'une quelconque des revendication 1 à 14, dans lequel, dans l'étape (f), l'analyse par chromatographie en phase gazeuse-spectrométrie de masse (GC-MS/MS) est effectuée par surveillance des réactions multiples (anglais : *multiple reaction monitoring MRM*).
